# EUROPEAN PATENT APPLICATION

(11) **EP 4 403 115 A2**
(43) Date of publication of application: **24.07.2024**
(21) Application number: 23220397.6
(22) Date of filing: 27.12.2023
(51) Int. Cl.: A61B 17/00, A61B 34/00, A61B 5/055, A61B 10/02, A61B 90/00

(54) **BIOPSY SYSTEM FOR USE IN MAGNETIC RESONANCE IMAGING SUITE**

(30) Priority: 28.12.2022 US 202263435620 P
(71) Applicant: Devicor Medical Products, Inc., Cincinnati, OH 45241 (US)
(72) Inventor: SHADIX, Peter, Cincinnati, Ohio, 45209 (US); NOCK, Andrew P., Dayton, Ohio, 45449 (US); MILLER, Eric J., Cincinnati, Ohio, 45212 (US); GRAHAM, Mark A., Cincinnati, Ohio, 45040 (US); WAGNER, Gary S., Independence, Kentucky, 41051 (US); SHUBERT, Jake, Bellevue, Kentucky, 41073 (US); FRIEDL, Mary, Covington, Kentucky, 41011 (US)
(74) Representative: Simmons & Simmons

(57) **Abstract**

A control module for use in a magnetic resonance imaging (MRI) guided biopsy procedure using an MRI coil includes a body and a plurality of MRI compatibility features. The body includes one or more ports configured to couple the control module to a biopsy device. The body further includes a display configured to output one or more biopsy device status indicators. The MRI compatibility features are configured to reduce an electromagnetic footprint of the control module.

## Description

### PRIORITY

This application claims priority to U.S. Provisional Patent App. No. 63/435,620, entitled "Biopsy System for Use in Magnetic Resonance Imaging Suite," filed on December 28, 2022, the disclosure of which is hereby incorporated by reference herein.

### BACKGROUND

Biopsy samples have been obtained in a variety of ways in various medical procedures using a variety of devices. Biopsy devices may be used under stereotactic guidance, ultrasound guidance, Magnetic Resonance Imaging (MRI) guidance, Positron Emission Mammography (PEM) guidance, Breast-Specific Gamma Imaging (BSGI) guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device.

Examples of such biopsy devices and biopsy system components are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pat. No. 7,442,171, entitled "Remote Thumbwheel for a Surgical Biopsy Device," issued October 8, 2008; U.S. Pat. No. 7,854,706, entitled "Clutch and Valving System for Tetherless Biopsy Device," issued December 1, 2010; U.S. Pat. No. 7,938,786, entitled "Vacuum Timing Algorithm for Biopsy Device," issued May 10, 2011; and U.S. Pat. No. 8,118,755, entitled "Biopsy Sample Storage," issued February 21, 2012; and U.S. Pat. No. 10,201,333, entitled "MRI Biopsy System," issued February 12, 2019. The disclosure of each of the above-cited U.S. Patents is incorporated by reference herein.

MRI guided biopsy procedures may involve unique operational constraints due to the strong magnetic field associated with the area proximate the MRI coil. The presence of this strong magnetic field may present particular constraints with respect to ferromagnetic objects because such objects may be attracted to the strong magnetic field. Additionally, electronic components may emit electromagnetic radiation that may interfere with sensitive components of associated with the MRI coil, thereby creating irregularities or image artifacts in images produced by the MRI coil. Thus, it may be desirable to isolate or otherwise segregate certain components from the MRI coil during an MRI guided biopsy procedure.

Isolation or segregation of components from the MRI coil may introduce usability challenges such as trip hazards, patient interaction challenges, and logistical challenges from moving between various components associated with the procedure. Thus, in some circumstances it may be desirable to move certain components closer to the MRI coil or in closer proximity with other components used in the procedure. Although some proximity of ferromagnetic objects relative to the MRI coil may be tolerated, the strength of the magnetic field and the specific distance separating the MRI coil from the ferromagnetic objects are factors that directly influence this tolerance.

While several systems and methods have been made and used for obtaining a biopsy sample, it is believed that no one prior to the inventor has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an example MRI biopsy system;
FIG. 2 depicts a top plan view of an example use of the MRI biopsy system of FIG. 1 within an example of a magnet room of an MRI suite;
FIG. 3 depicts a perspective view of a biopsy device of the MRI biopsy system of FIG. 1;
FIG. 4 depicts a perspective view of a foot switch of the MRI biopsy system of FIG. 1;
FIG. 5 depicts a perspective view of a control module of the MRI biopsy system of FIG. 1;
FIG. 6 depicts a detailed perspective view of the control module of FIG. 5;
FIG. 7 depicts perspective cutaway view of the control module of FIG. 5;
FIG. 8 depicts a detailed perspective cutaway view of the control module of FIG. 5; and
FIG. 9 depicts another perspective view of the control module of FIG. 5, the control module including a battery compartment with a door in an open configuration;
FIG. 10A depicts a cross-sectional view of a lock assembly for use in the battery compartment of FIG. 9, the lock assembly in an unlocked configuration;
FIG. 10B depicts another cross-sectional view of the lock assembly of FIG. 10A, the lock assembly in a locked configuration;
FIG. 11A depicts a cross-sectional view of another lock assembly for use in the battery compartment of FIG. 9, the lock assembly in an unlocked configuration;
FIG. 11B depicts another cross-sectional view of the lock assembly of FIG. 11A, the lock assembly in a locked configuration;
FIG. 12 depicts a schematic view of yet another lock assembly for use in the battery compartment of FIG. 9;
FIG. 13 depicts a schematic view of a signal processing system for use in connection with the MRI biopsy system of FIG. 1;
FIG. 14 depicts a schematic view of a user interface screen for use in connection with the MRI biopsy system of FIG. 1;
FIG. 15 depicts a schematic view of another user interface screen for use in connection with the MRI biopsy system of FIG. 1; and
FIG. 16 depicts a flowchart of an imaging mode algorithm for use in connection with the MRI biopsy system of FIG. 1.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Overview of Example MRI Biopsy System

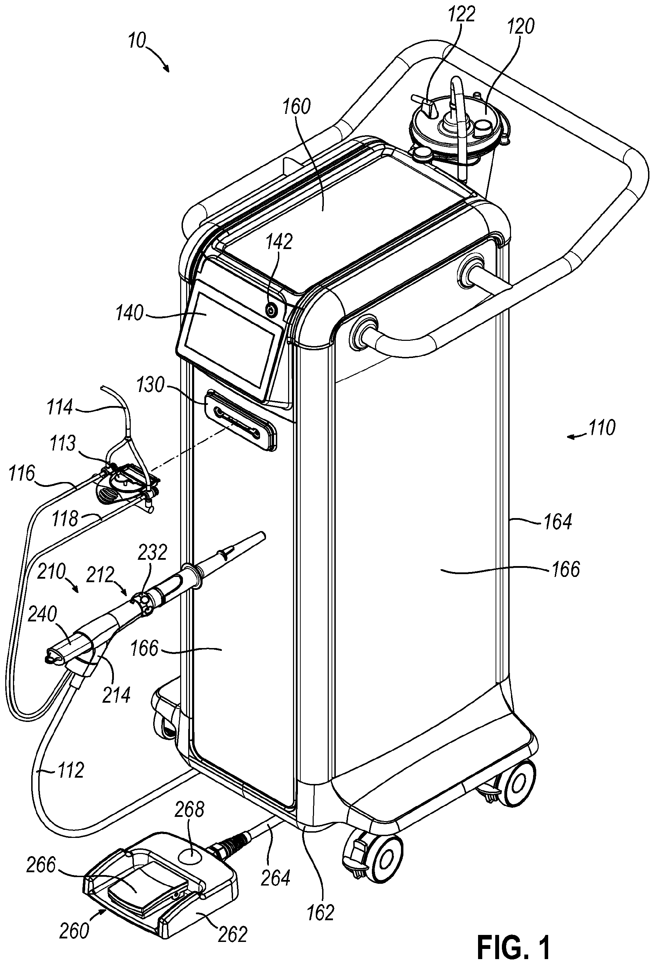

FIG. 1 shows an example of a Magnetic Resonance Imaging (MRI) compatible biopsy system (10) that may be used to collect one or more biopsy samples during an MRI guided biopsy procedure. MRI biopsy system (10) includes a control module (110). As will be described in greater detail below, control module (110) may be configured for use within a shielded room containing an MRI coil. Thus, control module (110) may include certain MRI compatibility features configured to reduce the electromagnetic footprint of control module (110). In other words, such MRI compatibility features may be configured to mitigate detrimental interaction with the strong magnetic field associated with the MRI coil and/or sensitive radio frequency (RF) signal detection sensors and/or antennas. A reduction of the electromagnetic footprint of control module (110) may include reducing electromagnetic radiation generated by one or more components of control module (110). A reduction of the electromagnetic footprint of control module (110) may further include reducing the propensity of control module (110) to be attracted or otherwise respond to a magnetic field. A plurality of specific examples of such MRI compatibility features will be described in greater detail below that may be readily incorporated into control module (110). As described in U.S. Pat. No. 6,752,768, which is hereby incorporated by reference in its entirety, a range of preprogrammed functionality may be incorporated into control module (110) to assist in collecting one or more tissue samples.

MRI biopsy system (10) further includes a biopsy device (210), and a foot switch (260) configured to control various functions of biopsy device (210). Control module (110) may be in communication with biopsy device (210) to control and/or power biopsy device (210) during use. In particular, control module (110) may be mechanically, electrically, and/or pneumatically coupled to biopsy device (210) so that components may be operated at various distances relative to the strong magnetic field and the sensitive RF receiving components of an MRI coil.

Control module (110) may include one or more cables (112) and/or tubes (114, 116, 118) coupling biopsy device (210) to control module (110). Such cables (112) and/or tubes (114, 116, 118) may be generally configured to communicate with biopsy device (210) to provide certain operational controls as will be described in greater detail below. In the present version, control module (110) includes a single cable (112) and a plurality of tubes (114, 116, 118), another various alternative numbers may be used.

In the present version, cable (112) may be configured to communicate control signals and cutter rotation/advancement motions respectively and may be connected to respective electrical and mechanical ports (not shown) in control module (110). Thus, cable (112) may be configured as a mechanical rotary drive cable, an electrical cable, or both. As will be described in greater detail below, cable (112) may additionally include certain features to promote use of control module (110) and/or biopsy device (210) proximate an MRI coil.

Vacuum assist may be provided by first vacuum tube (114) configured to communicate with an outlet port (122) of a vacuum canister (120) disposed on a portion of control module (110). A valve assembly (113) may be disposed between first vacuum tube (114) and biopsy device (210). A second vacuum tube (116) and third vacuum tube (118) extend from valve assembly (113) and couple to biopsy device (210). In this configuration, control module (110) may communicate vacuum, saline, atmospheric air, and/or other fluids to various portions of biopsy device (210) for use in collecting one or more tissue samples.

Control module (110) may further include a valve port (130) configured to receive valve assembly (113). In particular, control module (110) may include one or more motors (not shown) associated with valve port (130) and configured to drive valve assembly (113) when valve assembly (113) is disposed within valve port (130). In this configuration, valve port (130) may permit the collection of tubes (114, 116, 118) and valve assembly (113) (the collection of which may be referred to as a "tube set") to be entirely disposable. In some versions, any one or more of tubes (114, 116, 118) and valve assembly (113) may be configured and operable in accordance with the teachings of U.S. Patent No. 6,162,187, entitled "Fluid Collection Apparatus for a Surgical Device," issued December 19, 2000; or U.S. Patent No. 9,724,076, entitled "Biopsy Device Valve Assembly," issued August 8, 2017, the disclosure of which is incorporated by reference herein.

Control module (110) may further include a display (140) and a physical power button (142). Display (140) may be configured to control certain operational features of control module (110) and/or biopsy device (210), while power button (142) may be used to power control module (110) off and on, and/or wake control module (110) from a sleep state. Display (140) may further be configured to output certain control module (110) and/or biopsy device (210) status indicators. By way of example only, some such status indicators may include the level of vacuum supplied to biopsy device (210), a particular mode of operation associated with biopsy device (210), the position of components within biopsy device (210) (e.g., needle, cutter, tissue sample holder, etc.), and/or etc. In some versions, display (140) may receive operator input directly with display (140) being configured as a touchscreen. In addition, or in the alternative, display (140) may be associated with one or more buttons to facilitate receipt of input.

Of course, the above-described control module (110) is merely one example. Any other suitable type of control module (110) and associated components may be used. In some versions, control module (110) may be configured in accordance with any one or more of the teachings of US Pat. No. 10,201,333, entitled "MRI Biopsy System," issued on February 12, 2019, the disclosure of which is incorporated by reference herein. In other versions, control module (110) may instead be configured and operable in accordance with the teachings of U.S. Pub. No. 2008/0228103, entitled "Vacuum Timing Algorithm for Biopsy Device," published September 18, 2008, the disclosure of which is incorporated by reference herein. In still other versions, control module (110) may instead be configured and operable in accordance with the teachings of U.S. Patent No. 8,328,732, entitled "Control Module Interface for MRI Biopsy Device," issued December 11, 2012, the disclosure of which is incorporated by reference herein. Alternatively, control module (110) may have any other suitable components, features, configurations, functionalities, operability, etc. Other suitable variations of control module (110) and associated components will be apparent to those of ordinary skill in the art in view of the teachings herein.

Optionally, in some versions, MRI biopsy system (10) may include a localization fixture (no shown), and a patient support table (not shown). Such localization fixtures and patient support tables may be used to orient biopsy device (210) relative to a patient and the patient relative to the MRI coil. Such localization fixtures and patient support tables may additionally be used in combination with certain accessory components such as targeting sets including obturators and/or cannulas. Suitable localization fixtures, patient support tables, and/or targeting sets may be configured in accordance with any one or more of the teachings of US Pat. No. 10,201,333, entitled "MRI Biopsy System," issued on February 12, 2019, the disclosure of which is incorporated by reference herein. In other versions, suitable localization fixtures, patient support tables, and/or targeting sets may instead be configured and operable in accordance with the teachings of U.S. Pub. No. 2007/0255168, entitled "Grid and Rotatable Cube Guide Localization Fixture for Biopsy Device," published November 1, 2007, and incorporated by reference herein.

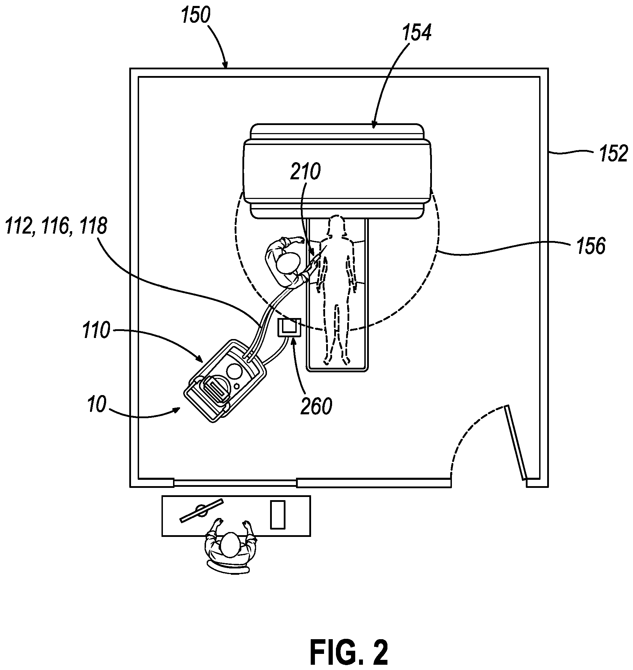

FIG. 2 shows an example of a magnet room (150) of an MRI suite, where MRI biopsy system (10) may be used to collect one or more tissue samples under MRI guidance from a patient. As can be seen, magnet room (150) may include a plurality of walls (152) defining a room or open space. Magnet room (150) may further include an MRI coil (154) configured for imaging tissue of a patient by generating and detecting strong electromagnetic fields. It should be understood that in some versions, walls (152) may include shielding or other forms of insulation to block electromagnetic radiation from interfering with MRI coil (154).

As will be described in greater detail below, MRI biopsy system (10) is configured for use entirely within magnet room (150). As best seen in FIG. 2, biopsy device (210) and foot switch (260) may be used proximate a patient to collection one or more tissue samples. Meanwhile, control module (110) may also be positioned within magnet room (150) proximate an operator, but outside a gauss zone (156).

In the present version, gauss zone (156) is an imaginary arc a predetermined radius relative to MRI coil (154), outside of which control module (110) may be used. As will be understood, the strong electromagnetic field generated by MRI coil (154) may interact with components near MRI coil (154). For instance, components including ferromagnetic materials may be attracted to MRI coil (154). Similarly, the same or other components may interact with MRI coil (154) itself by generating electromagnetic radiation that may interfere with sensitive electromagnetic detection components within MRI coil (154). In other interactions, one or more functions of the ferromagnetic materials may be impaired by the strong electromagnetic field generated by MRI coil (154), thereby causing one or more electrical or mechanical malfunctions with control module (110). Such interactions may generally be a function of distance relative to MRI coil (154), among other things. In particular, control module (110) may include several components including both ferromagnetic materials as well as components configured to generate electromagnetic radiation (either intentionally or incidentally). Thus, gauss zone (156) shown in FIG. 2 is for use of control module (110) within magnet room (150) at a distance where any interactions between MRI coil (154) and control module (110) would be within acceptable levels. Of course, similar gauss zones may also be depicted for other components such as biopsy device (210) or foot switch (260). However, as will be described in greater detail below, biopsy device (210) and foot switch (260) are configured for minimal interaction with MRI coil (154) and therefore have substantially smaller respective gauss zones.

### II. Example of MRI Compatible Biopsy Device

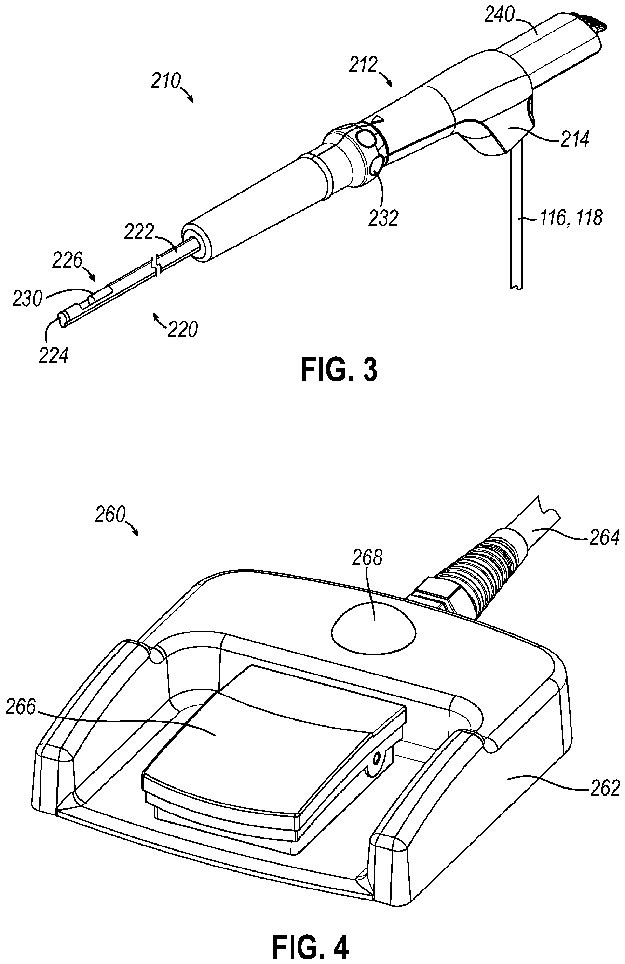

Biopsy device (210) is shown in greater detail in FIG. 3. Biopsy device (210) is generally configured as a core needle biopsy device with certain features to promote use of biopsy device (210) proximate an MRI coil such as MRI coil (154). Biopsy device (210) includes a probe (212), a needle assembly (220), and a tissue sample holder (240). Probe (212) is generally configured for grasping by an operator with one or more hands for manipulation of needle assembly (220). Probe (212) is further configured to house various operational components associated with needle assembly (220) such as drive components such as gears, shafts, and/or etc. and fluid control components such as tubes, conduits and/or etc. Such operational components may include entirely MRI compatible materials such as polymers, plastics, rubbers, ceramics, and/or non-magnetic metals including nonferrous metals (e.g., titanium, aluminum, brass, copper bronze, alloys thereof, and/or etc.) or steel grades with limited ferrite phase.

Additionally, probe (212) may omit onboard power sources and/or electronics to further promote MRI compatibility. Instead of including onboard power sources and/or electronics, probe (212) may be powered entirely by external sources. For instance, in the present version, power may be provided to probe (212) by cable (112) and/or tubes (114, 116, 118). Suitable power sources may therefore be isolated from probe (212) and instead be incorporated into control module (110). Accordingly, probe (212) includes a cable port (214) to permit coupling of probe (212) to cable (112). Meanwhile, tubes (116, 118) may be integrated into probe (212) as shown, or alternatively separately coupled to probe (212) by one or more coupling features or ports.

Needle assembly (220) extends distally from probe (212). Needle assembly (220) is generally configured to sever one or more tissue sample from a patient and communicate such severed tissue samples through probe (212) to tissue sample holder (240). In the present version, needle assembly (220) includes dense rigid materials such as metals and/or ceramics. In some versions, needle assembly (220) may include entirely MRI compatible materials such as non-magnetic metals including nonferrous metals (e.g., titanium, aluminum, brass, copper bronze, alloys thereof, and/or etc.), or steel grades with limited ferrite phase, and/or ceramics. In other version, needle assembly (220) may include some ferrous metals such as stainless steel. In some circumstances, at least some ferrous metals may be tolerated due to the relatively low mass of needle assembly (220).

Needle assembly (220) includes an outer cannula (222) and a cutter (230). Outer cannula (222) defines a distal tip (224) and a lateral aperture (226) proximate distal tip (224). In the present version, distal tip (224) is of a blunt configuration. Such a blunt configuration may be desirable for circumstances where needle assembly (220) is used in combination with the targeting set described above because the targeting set may include dedicated features for tissue penetration. In other versions, distal tip (224) may include a sharp configuration configured to penetrate tissue.

Cutter (230) may be disposed within a hollow interior of outer cannula (222). Cutter (230) is generally configured to translate and rotate within the hollow interior of outer cannula (222) relative to lateral aperture (226) to sever a tissue sample from tissue prolapsed into lateral aperture (226). Although not shown, it should be understood that cutter (230) may be hollow to facilitate transport of severed tissue samples through cutter (230) under vacuum provided by tubes (116, 118).

Probe (212) further includes a needle rotation feature (232). Needle rotation feature (232) is generally configured to rotate outer cannula (222) of needle assembly (220) about the longitudinal axis of needle assembly (220). Rotation of outer cannula (222) may be desirable in some versions to permit adjustment of the orientation of lateral aperture (226). For instance, outer cannula (222) may be rotated to orient lateral aperture (226) in a variety of clock positions so that lateral aperture (226) may be used to collect tissue samples at a variety of positioned around the perimeter of outer cannula (222). Although the present version includes needle rotation feature (232), in other versions, needle rotation feature (232) is entirely optional and may be omitted.

Tissue sample holder (240) extends proximally from probe (212). Tissue sample holder (240) is generally configured to receive and contain tissue samples severed by cutter (230). It should be understood that tissue sample holder (240) may include a variety of configurations. For instance, in some versions, tissue sample holder (240) may include a single tray for collection of tissue samples in bulk. In other versions, tissue sample holder (240) may include one or more trays with each tray having a plurality of strips for collection of tissue samples individually. In yet other versions, tissue sample holder (240) is optional and may be omitted entirely. Still other configurations of tissue sample holder (240) may be apparent to those of ordinary skill in the art in view of the teachings herein.

By way of example only, biopsy device (210) may be configured in accordance with at least some of the teachings of U.S. Pub. No. 2010/0160824, the disclosure of which is incorporated by reference herein; U.S. Patent Pub. No. 2013/0144188, entitled "Biopsy Device with Slide-In Probe," published June 6, 2013, the disclosure of which is incorporated by reference herein; U.S. Patent Pub. No. 2013/0324882, entitled "Control for Biopsy Device," published December 5, 2013, the disclosure of which is incorporated by reference herein; U.S. Patent Pub. No. 2014/0039343, entitled "Biopsy System," published February 6, 2014, the disclosure of which is incorporated by reference herein; and/or U.S. Patent App. No. 14/469,761, entitled "Tissue Collection Assembly for Biopsy Device," filed August 27, 2014, the disclosure of which is incorporated by reference herein.

### III. Example of MRI Compatible Foot Switch

Foot switch (260) is generally configured to control operation of biopsy device (210) using the foot of an operator. In this configuration, biopsy device (210) may be grasped using a single hand, permitting use of the other hand for manipulation of accessory components or needle rotation feature (232). Additionally, foot switch (260) is configured for MRI compatibility by including minimal ferrous materials.

As best seen in FIG. 4, foot switch (260) includes a base (262), a cable (264) extending from base (262), and one or more user input features (266, 268). Base (262) is generally configured to be positioned on a floor such as the floor of magnet room (150) described above. Due to the positioning of base (262) on a floor, base (262) may have a relatively large size to promote use via a foot and to improve the visibility of foot switch (260). Base (262) may also optionally include weights or relatively dense materials to help maintain foot switch (260) in a predetermined position on floor. Additionally, base (262) may optionally include one or more elastomeric feet or other stabilizing features positioned on a bottom surface of base (262). Use of such elastomeric feet may be desirable in some versions to maintain foot switch (260) in a stable position on the floor.

Base (262) is further configured to house components associated with user input features (266, 268). Thus, a portion of base (262) may be hollow. To promote MRI compatibility, the structure of base may include MRI compatible materials such as polymers, plastics, rubbers, ceramics, and/or non-magnetic metals including nonferrous metals (e.g., titanium, aluminum, brass, copper bronze, alloys thereof, and/or etc.) or steel grades with limited ferrite phase.

User input features (266, 268) of the present version include a foot pedal (266) and a push button (268). Both foot pedal (266) and push button (268) are configured to act as switches such that actuation of foot pedal (266) or push button (268) may communicate a signal to control module (110). Both foot pedal (266) and push button (268) may additionally be oversized to promote actuation by a foot of an operator.

To promote MRI compatibility, foot pedal (266) and push button (268) may include predominantly MRI compatible materials such as polymers, plastics, rubbers, ceramics, and/or non-magnetic metals including nonferrous metals (e.g., titanium, aluminum, brass, copper bronze, alloys thereof, and/or etc.) or steel grades having limited ferrite phase. However, due to the position of foot switch (260) on a floor where electromagnetic fields may be weaker, at least some ferrous materials may be tolerated where beneficial. For instance, components used to permit foot pedal (266) and push button (268) as switches may include at least some ferromagnetic materials. Thus, various electronic switch components may be used to permit foot pedal (266) and push button (268) to act as switches. However, where possible, various electronic components may be segregated to control module (110) and in communication with foot switch (260) via cable (264).

### IV. Example of Control Module for Use in MRI Suite

As described above, it may be desirable in some circumstances to position control module (110) within a portion of an MRI suite such as magnet room (150) described above. When control module (110) is positioned outside magnet room (150), certain operational challenges may occur under some circumstances. For instance, the remote nature of control module (110) relative to the clinician performing the biopsy procedure may contribute to challenges with making operational adjustments to control module (110), particularly mid-biopsy procedure. Additionally, under some circumstances, the length of cable (112) and/or tubes (116, 118) may contribute to operational challenges. For instance, cable (112) and/or tubes (116, 118) may introduce operational delays or inefficiencies when closing the door of magnet room (150) when performing an image scan, which may require retraction of cable (112) and/or tubes (116, 118). Cable (112) and/or tubes (116, 118) may also present a trip hazard under some circumstances. The relatively long length of cable (112) may also lead to unintentional movement of cable (112) during a biopsy procedure due to internal torque within cable (112).

In view of the discussion above, it may be desirable under some circumstances to use control module (110) within a portion of an MRI suite such as magnet room (150). However, as noted above, the presence of an MRI coil such as MRI coil (154) may lead to undesirable effects due to the electromagnetic field generated by the MRI coil. Accordingly, it may be desirable to include certain features within control module (110) to promote the use of control module (110) within magnet room (150) with limited interaction between control module (110) and the electromagnetic field generated by MRI coil (154).

### A. Example Control Module Features to Enhance MRI Compatibility

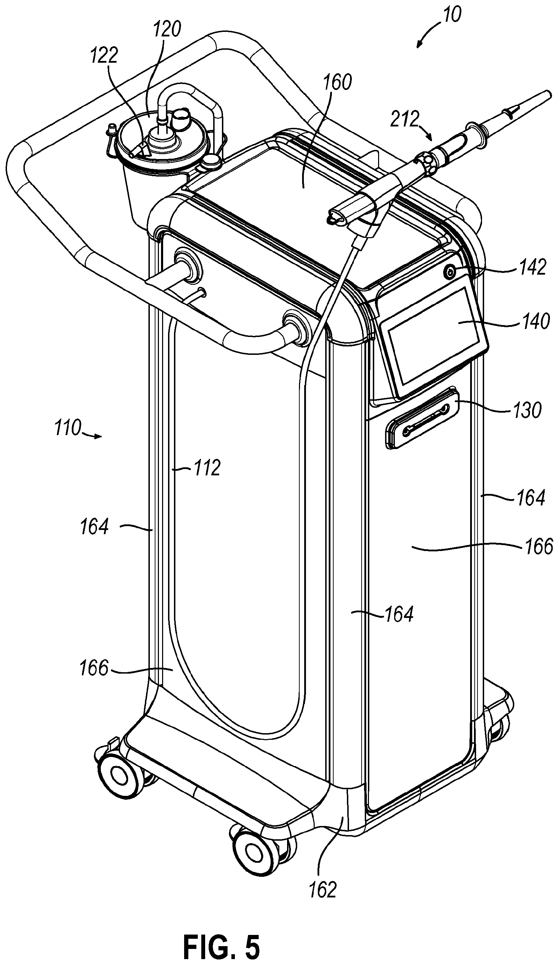

FIG. 5 shows cable (112) in greater detail. As can be seen, cable (112) of the present version is relatively short. In this relatively short configuration, cable (112) may be more resistant to movement arising from internal torque within cable (112). In the present version, "relatively short" corresponds to a length of about 2 times the height of control module (110) or less. In absolute terms, "relatively short" may correspond to about 8 feet or less in some versions, about 6 feet or less in other versions, about 4 feet or less in yet other versions, or between about 7 and 4 feet in still other versions.

The particular structure of cable (112) may also be configured to promote use within magnet room (150). For instance, the diameter of cable may be minimized to reduce interaction with the electromagnetic filed generated by MRI coil (154) and to reduce the propensity of cable (112) kinking. Additionally, the particular materials used may be configured to reduce interaction with the electromagnetic field generated by MRI coil (154). In some versions, at least a portion of cable (112) may include phosphor bronze or other non-magnetic metals including nonferrous metals (e.g., titanium, aluminum, brass, copper bronze, alloys thereof, and/or etc.) or steel grades having limited ferrite phase.

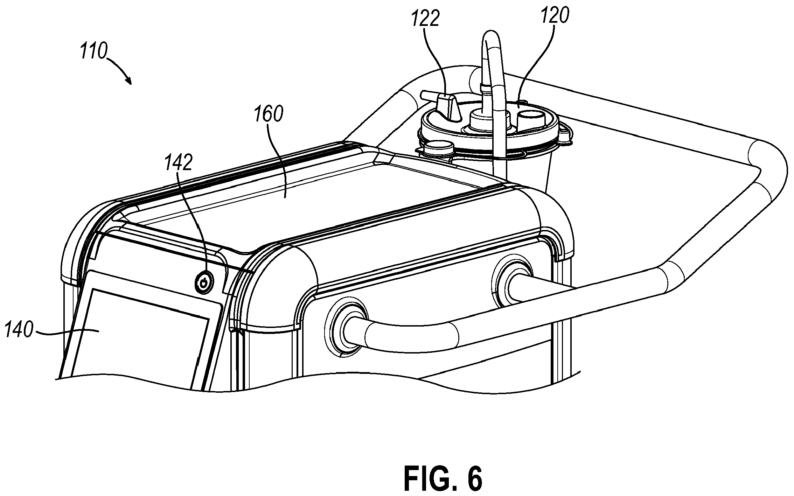

Control module (110) may further include a work area (160). As best seen in FIG. 6, work area (160) of the present version is defined as a recessed contour in an upper surface of control module (160). Work area (160) is generally configured to provide an area for placement of biopsy device (210) and/or other features associated with a biopsy procedure such as a targeting set. Work area (160) may be desirable to eliminate the need for a separate tray or other similar features that may include ferrous materials. In the present version, work area (160) is configured as a depression or indentation in the upper surface of control module (110). In other versions, work surface (160) may be configured as a separate planar component similar to a shelf. In still other versions, work surface (160) is entirely optional and may be omitted entirely.

Although not shown, in some examples, work area (160) is configured to receive certain accessory components. By way of example only, in some examples an optional tray can be removably received within work area (160) or a portion thereof. Such an optional tray may be desirable to maintain the sterility of work area and/or other portions of control module (110). In some examples, such an optional tray can be configured to include a plurality of discrete sections or segments separated by walls indentations, or other features. For instance, in one example one section is sized similarly to a mayo stand, while another section is sized smaller, but with a lower floor position. Of course, work area (160) may be configured for use with other suitable trays or accessory components as will be appreciated by those of ordinary skill in the art in view of the teachings herein.

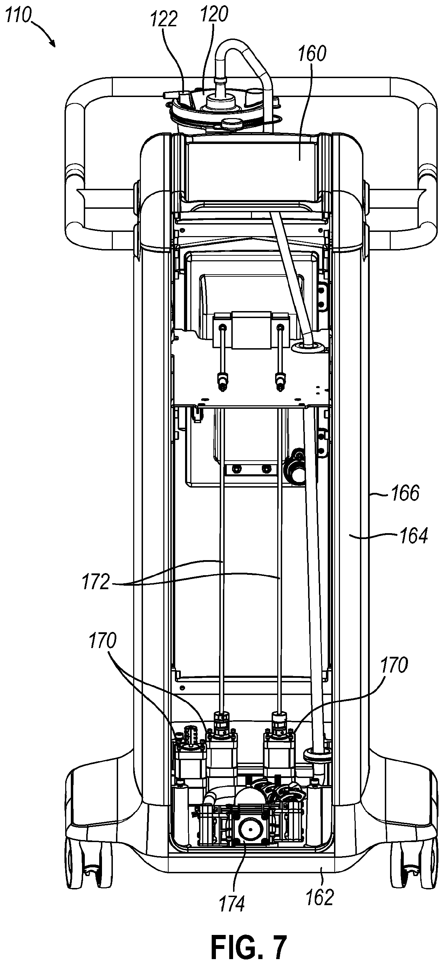

As best seen in FIG. 7, control module (110) includes a physical structure configured to promote use in a portion of an MRI suite similar to magnet room (150) described above. In particular, control module (110) includes a base (162), structural members (164), and body panels (166) of all MRI compatible materials. For instance, base (162) and structural members (164) may include non-ferrous metals such as titanium, aluminum, brass, copper bronze, alloys thereof, and/or etc. Base (162) and structural members (164) may also include non-magnetic metals, which may encompass non-ferrous metals as well as certain grades of steel with limited ferrite phase (e.g., austenitic stainless steels). The use of non-ferrous or non-magnetic metals for base (162) and structural members (164) may be desirable in the present version of other MRI compatible materials to provide enhanced rigidity and durability. Meanwhile, body panels (166) may include other MRI compatible materials such as plastic. The use of plastic for body panels (166) in the present version may be desirable to provide light weight, ease of manufacturability, color customization, and/or etc.

In the present version, base (162) and structural members (164) may define the general shape of control module (110), while body panels (166) may fill spaces between base (162) and structural members (164) to enclose the interior of control module (110). Thus, base (162) may form a generally rectangular platform for mounting various components thereon, while structural members (164) may extend upwardly from base (162) to define the rectangular structure of control module (110). Base (162) may further support other associated components such as wheels. Body panels (166) may then be attached to structural members (164) to fill the spaces between base (162) and structural members (164).

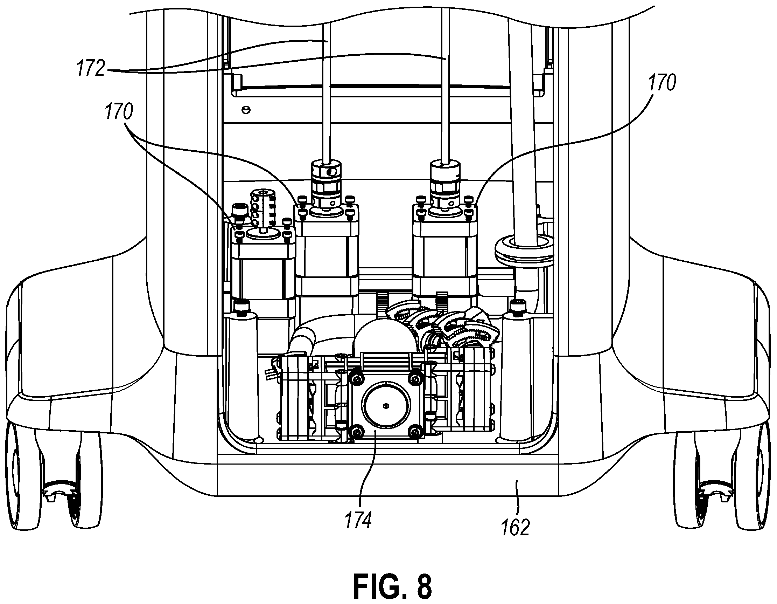

As best seen in FIGS. 7 and 8, the internal components of control module (110) are generally configured to promote MRI compatibility. In particular, control module (110) is configured such that high density ferrous components may be oriented towards a bottom portion of control module (110). In the present version, this configuration includes one or more motors (170) and one or more vacuum pumps (174), which are positioned proximate base (162). In this position, motors (170) and vacuum pumps (174) are generally oriented closer to the floor where the electromagnetic field generated by an MRI coil similar to MRI coil (154) is lower. Thus, by positioning motors (170) and vacuum pumps (174) closer to base (162), control module (110) may be positioned closer to an MRI coil similar to MRI coil (154) without having adverse effects on the operation of the MRI coil or vice-versa. In some versions, "closer to base (162)" may include any high-density ferrous components being positioned below one half the total heigh of control module (110). In other versions, "closer to base (162)" may include any high-density ferrous components being positioned below one quarter of the total height of control module (110). In yet other versions, "closer to the base (162)" may include any high-density ferrous components being positioned below one eighth of the total height of control module (110).

Due to the positioning of motors (170) and vacuum pumps (174), control module (110) may further include certain communication features configured to facilitate communication of motors (170) and/or vacuum pumps (174) with other portions of control module (110). For instance, in the present version, control module (110) may include one or more linkages (172) corresponding to each motor (170) to communicate rotatory motion from each respective motor (170) to another portion of control module (110). Linkages (172) may generally include one or more elongate shafts of MRI compatible material extending through the interior of control module (110). To facilitate communication of rotary motion to other components, linkages (172) may also be associated with other drive components such as bearings, gears, screws, and/or etc. Similarly, although not shown, it should be understood that vacuum pumps (174) may be in communication with elongate tubes, conduits, and/or pipes to communicate vacuum from one portion of control module (110) to another.

### B. Example Battery Control Features

In some versions, it may be desirable to power control module (110) using battery power rather than wired power via a wall outlet. Such a configuration may be desirable for a variety of reasons. For instance, wall outlets may not always be present within a portion of an MRI suite such as magnet room (150) described above. Additionally, the use of battery power may permit control module (110) to be entirely operated by direct current rather than alternating current, which may reduce electromagnetic noise. Furthermore, the use of battery power may improve usability by promoting tetherless mobility.

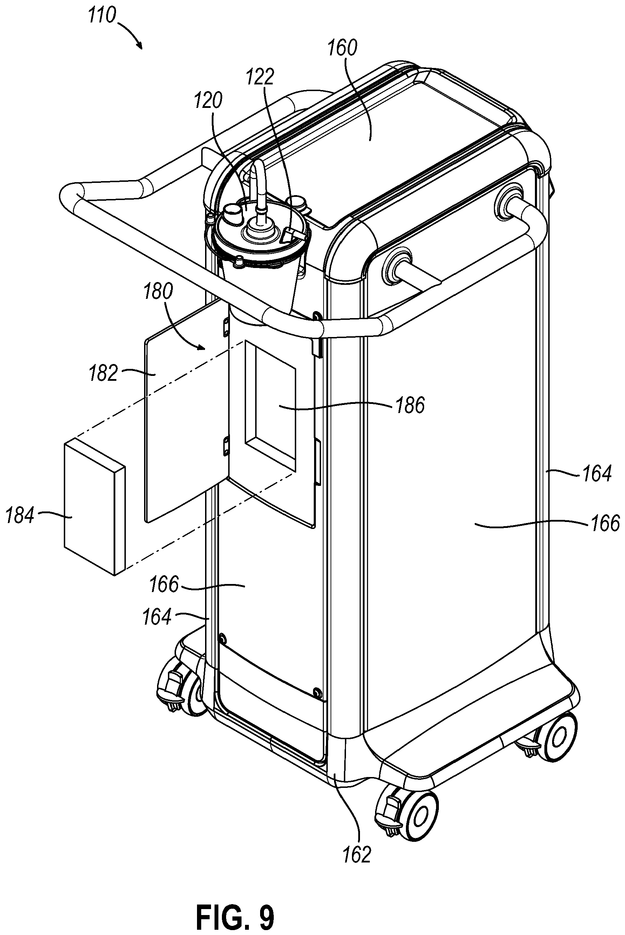

FIG. 9 shows an example of a battery compartment (180) that may be integrated into a portion of control module (110). As can be seen, battery compartment (180) includes a door (182) and a battery receiver (186) disposed within battery compartment (180) behind door (182). Door (182) is configured to pivot between an open and closed configuration to selectively enclose battery receiver (186). As will be described in greater detail below, in some versions of battery compartment (180), door (182) may be equipped with a lock to selectively lock door (182) in the closed configuration, thereby limiting access to battery compartment (180). In addition, or in the alternative, door (182) can be associated with an alarm or other indicator. Such an alarm or indicator can be configured to prevent changing of battery (184) while MRI coil (154) is on.

Battery receiver (186) is configured to receive a battery (184). In the present version, battery receiver (186) is configured as a pocket with an open top portion. In this configuration, battery receiver (186) may have a drop-in configuration such that battery (184) may be dropped into battery receiver (186). In other versions, battery receiver (186) may take on a variety of forms configured to receiver battery (184) such as an entirely open cavity with one or more latch features, a bottom insertion-style cavity, and/or etc. Although not shown, it should be understood that the interior of battery receiver (186) may include one or more electrical contacts configured to communicate electrical power between battery (184) to control module (110).

In some examples, battery (184) is optionally associated with one or more level indicators. Such level indicators can be integrated into battery (184) and/or one or more portions of control module such as dedicated lights and/or various user interface screens (500, 550) described in greater detail below. In some examples, level indicators may be configured as a red-yellow-green stoplight or percentage readout to provide a user with an indicator of the remaining battery power within battery (184). In addition or in the alternative, level indicators can include a counter to indicate the number of biopsy sampling cycles remaining on a given charge. Regardless, on or more functions of such level indicators can be modified based on the specific age of battery (184) to account for performance differences as battery (184) ages.

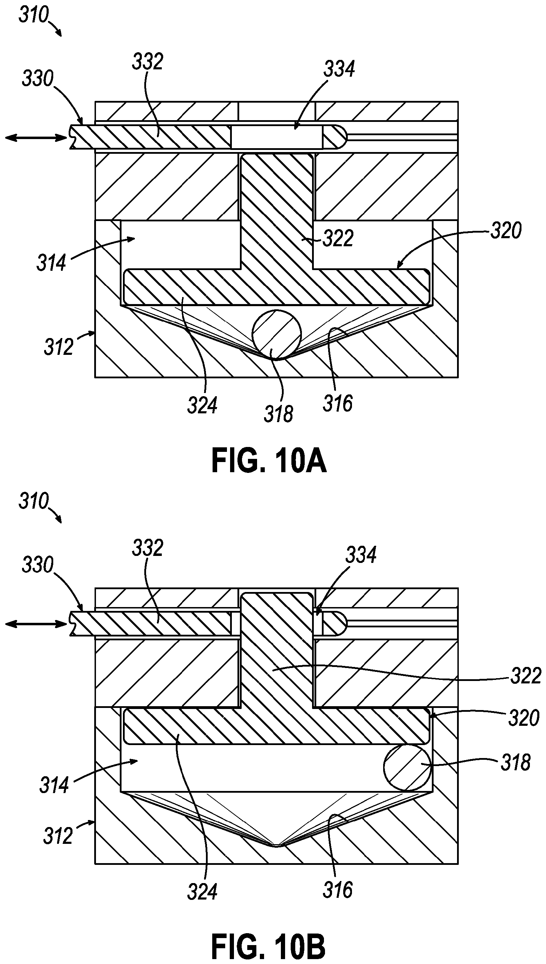

FIGS. 10A through 12 show a variety of lock assemblies (310, 340, 370) that may be used in combination with door (182) described above. Lock assemblies (310, 340, 370) are generally configured to selectively lock door (182) in response to a variety of operational conditions such as the detection of an electromagnetic field. Such a locking feature in combination with door (182) may be desirable to limit access to battery (184). For instance, during use of an MRI coil such as MRI coil (154) described above, the strong electromagnetic field associated with the MRI coil may attract or otherwise influence battery (184) due to the presence of ferromagnetic materials contained within battery (184). Control module (110) is configured so that use of control module (110) may occur with battery (184) present even when the MRI coil is on and thus producing the strong electromagnetic field. However, if battery (184) were removed from control module (110) during operation, the relative position between battery (184) and the MRI coil could be reduced to an unacceptable level, resulting in undesirable consequences from battery (184) being attracted to MRI coil. Thus, by locking door (182) and controlling access to battery (184), battery (184) may be used without being inadvertently drawn to the MRI coil.

FIG. 10A shows an example lock assembly (310) with a gauss-based lock mechanism. Lock assembly (310) is generally configured to respond to electromagnetic fields at a predetermined strength level to automatically lock door (182) of battery compartment (180). Lock assembly (310) of the present version includes a lock body (312) housing an actuation member (318), a locking lever (320) (also referred to as toggle, or locking member), and a lock catch (330). Lock body (312) defines a hollow interior (314) having a conical ramped surface (318) proximate the bottom of lock body (312). As will be described in grater detail below, hollow interior (314) is configured to receive various parts of lock assembly (310) such as actuation member (318) and locking lever (320).

Actuation member (318) is generally movable within lock body (312) in response to the presence of an electromagnetic field to change the state of lock assembly (310) between an unlocked configuration and a locked configuration. In particular, actuation member (318) is generally configured to respond to an electromagnetic field. In the present version, such responsiveness may be achieved by actuation member (318) including a ferrous or magnetic material. Actuation member (318) also defines a round or spherical shape to promote ease of movement within lock body (312) along features such as ramped surface (316).

Locking lever (320) is positioned above ramped surface (316) and actuation member (318). Locking lever (320) is generally cylindrical and includes a lock portion (322) and an engagement portion (324). In the present version, lock portion (322) generally defines a smaller diameter relative to engagement portion (324). In other versions, the diameters of lock portion (322) and engagement portion (324) may be varied such that the diameter of lock portion (322) may be larger than the diameter of engagement portion (324). Alternatively, in other versions, the diameter of lock portion (322) may be substantially similar to the diameter of engagement portion (324).

Lock catch (330) is movable within a portion of lock body (312) such as a channel or other conduit and is generally configured to be locked in position by one or more portions of lock assembly (310) to hold door (182) of battery compartment (180) in a closed configuration. Thus, although not shown, it should be understood that lock catch (330) may be secured to a portion of door (182), while lock body (312) may be secured to another portion of control module (110). Alternatively, in some versions, lock body (312) may be secured to door (182), while lock catch (330) may be secured to another portion of control module (110).

Lock catch (330) includes an attachment portion (332) and a lock opening (334) disposed proximate one end of attachment portion (332). Attachment portion (332) is generally configured to be fastened to door (182) or another feature of control module (110). Meanwhile, lock opening (334) is configured to selectively engage a portion of lock assembly (310) such as locking lever (320) to hold lock catch (330) in the locked configuration.

FIGS. 10A and 10B show an example use of lock assembly (310) for locking door (182). In particular, FIG. 10A shows lock assembly (310) in the unlocked configuration. In this configuration, the electromagnetic field proximate lock assembly (310) may be relatively low or not detectable. In some circumstances, this condition may correspond to an MRI coil being off, on standby, or otherwise not imaging. Because of the relatively low electromagnetic field, gravity may act on actuation member (318) pulling actuation member (318) downwardly along ramped surface (316) of lock body (312) to the vertex of ramped surface (316).

With actuation member (318) pulled downwardly under the force of gravity, locking lever (320) may likewise be pulled downwardly under the force of gravity toward ramped surface (316) and away from lock catch (330). With locking lever (320) moved away from lock catch (330), lock portion (322) may be disengaged from lock opening (334) of lock catch (330), thereby permitting free movement of lock catch (330) relative to lock body (312).

In the presence of an electromagnetic field, lock assembly (310) may move to the locked configuration automatically. As best seen in FIG. 10B, the presence of the electromagnetic field may attract actuation member (318) upwardly, overcoming the force of gravity. Actuation member (318) may then travel up ramped surface (316) into contact with engagement portion (324) of locking lever (320). As a result, actuation member (318) may push locking lever (320) upwardly to engage lock portion (322) with lock opening (334) of lock catch (330). Lock catch (330) may then be held in position relative to lock body (312), thereby holding door (182) closed.

It should be understood that the particular characteristics of actuation member (318) may be modified as needed so that actuation member (318) may be responsive to a particular electromagnetic field. For instance, to calibrate actuation member (318) for a relatively low or weak electromagnetic field, the particular amount of ferrous material included with actuation member (318) may be increased. Similarly, the weight of actuation member (318) may be reduced to change the amount of force required to overcome the force of gravity. In other versions, a combination of the amount of ferrous material and the weight of actuation member (318) may be adjusted in combination. Of course, to calibrate actuation member (318) for a relatively high or strong electromagnetic field, opposite but similar characteristics may be changed.

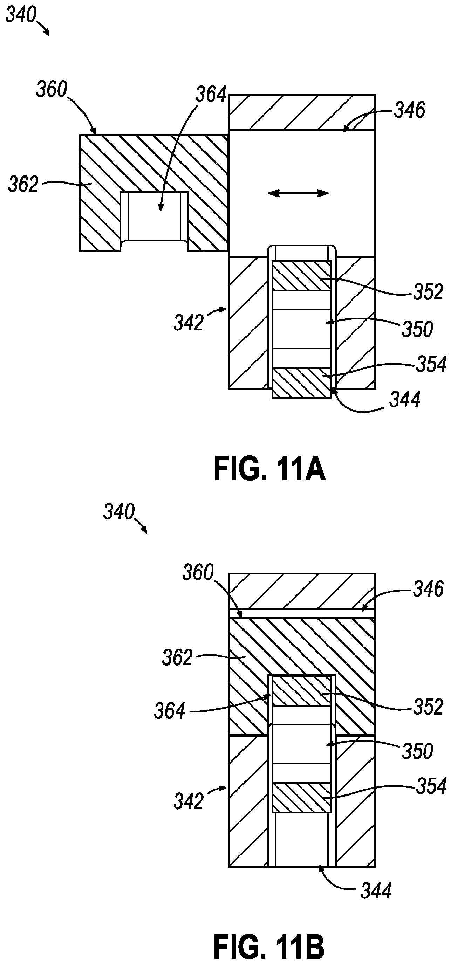

FIG. 11A shows another example lock assembly (340) with a gauss-based lock mechanism. Lock assembly (340) is generally configured to respond to electromagnetic fields at a predetermined strength level to automatically lock door (182) of battery compartment (180). Lock assembly (340) of the present version includes a lock body (342) housing a locking lever (350) (also referred to as toggle, or locking member), and a lock catch (360). Lock body (342) defines a lock channel (344) and a catch channel (348) extending through at least a portion of lock body (342). Specifically, lock channel (344) generally extends upwardly from a bottom surface of lock body (342) to intersect with catch channel (348). Meanwhile, catch channel (348) extends horizontally or perpendicularly relative to lock channel (344) from one side of lock body (342) to another.

Unlike lock assembly (310) described above, lock assembly (340) of the present version omits a separate structure similar to actuation member (318) described above. Instead, features corresponding to actuation member (318) may be incorporated into locking lever (350). In particular, locking lever (350) is generally movable within lock body (342) in response to the presence of an electromagnetic field to change the state of lock assembly (340) between an unlocked configuration and a locked configuration. In the present version, responsiveness of locking lever (350) to an electromagnetic field may be achieved by one or more portions of locking lever (350) including a ferrous or magnetic material.

Locking lever (350) is positioned within lock channel (344) of lock body (342) and is configured to move up and down within lock body (342). Locking lever (350) is generally rectangular or cylindrical and includes a lock portion (352) and an engagement portion (354). In the present version, lock portion (352) generally substantially similar to engagement portion (354) but positioned above engagement portion (354). In this configuration, engagement portion (354) may be configured to locate locking lever (350) within lock channel (344), while lock portion (352) may be configured to selectively engage one or more portions of lock catch (360) to move lock assembly (340) between a locked configuration and an unlocked configuration.

Lock catch (360) is movable within a portion of lock body (342) such as a catch channel (346) and is generally configured to be locked in position by one or more portions of lock assembly (340) to hold door (182) of battery compartment (180) in a closed configuration. Thus, although not shown, it should be understood that lock catch (360) may be secured to a portion of door (182), while lock body (342) may be secured to another portion of control module (110). Alternatively, in some versions, lock body (342) may be secured to door (182), while lock catch (360) may be secured to another portion of control module (110).

Lock catch (360) includes an attachment portion (362) and a lock opening (364) extending into the bottom surface of lock catch (360). Attachment portion (362) is generally configured to be fastened to door (182) or another feature of control module (110). Meanwhile, lock opening (364) is configured to selectively engage a portion of lock assembly (340) such as locking lever (350) to hold lock catch (360) in the locked configuration.

FIGS. 11A and 11B show an example use of lock assembly (340) for locking door (182). In particular, FIG. 11A shows lock assembly (340) in the unlocked configuration. In this configuration, the electromagnetic field proximate lock assembly (340) may be relatively low or not detectable. In some circumstances, this condition may correspond to an MRI coil being off, on standby, or otherwise not imaging. Because of the relatively low electromagnetic field, gravity may act on locking lever (350) pulling locking lever (350) downwardly within lock channel (344) toward the bottom of lock body (342) and away from lock channel (344) and lock catch (360).

With locking lever (350) pulled downwardly under the force of gravity, locking lever (320) may be pulled away from lock catch (360). As a result, lock portion (352) may be disengaged from lock opening (364) of lock catch (360), thereby permitting free movement of lock catch (360) relative to lock body (342) within catch channel (346).

In the presence of an electromagnetic field, lock assembly (340) may move to the locked configuration automatically. As best seen in FIG. 11B, the presence of the electromagnetic field may attract locking lever (350) upwardly, overcoming the force of gravity. Locking lever (350) may then travel upwardly within lock channel (344) of lock body (342) and into engagement with lock opening (364) of lock catch (360). Lock catch (360) may then be held in position relative to lock body (342), thereby holding door (182) closed.

It should be understood that the particular characteristics of locking lever (350) may be modified as needed so that locking lever (350) may be responsive to a particular electromagnetic field. For instance, to calibrate locking lever (350) for a relatively low or weak electromagnetic field, the particular amount of ferrous material included with locking lever (350) may be increased. Similarly, the weight of locking lever (350) may be reduced to change the amount of force required to overcome the force of gravity. In other versions, a combination of the amount of ferrous material and the weight of locking lever (350) may be adjusted in combination. Of course, to calibrate locking lever (350) for a relatively high or strong electromagnetic field, opposite but similar characteristics may be changed.

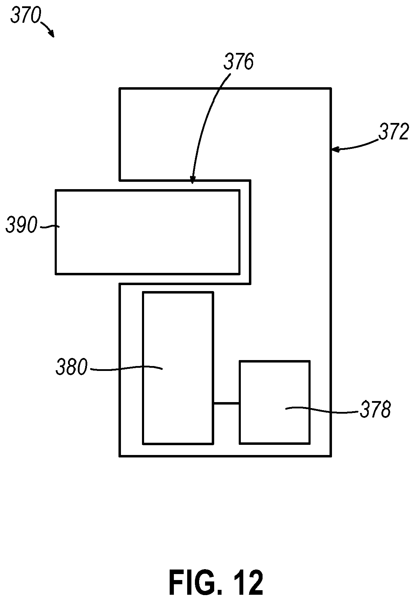

FIG. 12 shows another example of a lock assembly (370) similar to lock assemblies (310, 340) described above. As with lock assemblies (310, 340), lock assembly (370) of the present version is generally configured to automatically lock door (182) when the presence of an electromagnetic field is detected. However, unlike lock assemblies (310, 340), lock assembly (370) of the present version includes one or more electronic features to facilitate locking of door (182). Such electronic features may be desirable in some circumstances to increase the flexibility or adaptability of lock assembly (370). Such electronic features may further be desirable to integrate the function of lock assembly (370) with other functions of control module (110).

Lock assembly (370) of the present version includes a lock body (372) housing a gauss detector (378), a lock mechanism (380), and a lock catch (390). Lock body (372) defines a catch channel (376) extending through at least a portion of lock body (372). Specifically, catch channel (376) extends horizontally within lock body (372) from one side of lock body (372). In this configuration, catch channel (376) is generally configured to receive lock catch (390).

Gauss detector (378) is generally configured to respond to the presence of an electromagnetic field by communicating a single to other components of lock assembly (370) and/or control module (110). In some versions, the signal produced by gauss detector (378) may be proportional to the electromagnetic field present. In other versions, the signal produced by gauss detector (378) may be binary, such that one signal is communicated when an electromagnetic field above a predetermined threshold is detected and another signal is communicated with an electromagnetic field is below the predetermined threshold. Although a signal predetermined threshold may be used, in some versions, the predetermined threshold may be adjustable to customize operation of gauss detector (378) for specific operational environments.

Although gauss detector (378) is shown as being disposed within lock body (372), it should be understood that in other versions, gauss detector (378) may be disposed in other physical locations. For instance, in some versions, gauss detector (378) may be disposed in one or more portions of control module (110) where detection of an electromagnetic field may have a closer relationship with the physical position of control module within a portion of an MRI suite such as magnet room (150) described above. Such alternative positions for gauss detector (378) may be desirable in configurations where gauss detector (378) is integrated with other functions of control module (110) such as detecting the position of control module relative to an MRI coil such as MRI coil (154) described above.

Gauss detector (378) is in communication with lock mechanism (380). In the present version, lock mechanism (380) is configured to respond to one or more signals generated by gauss detector (378) to initiate a lock function. For instance, in some versions, lock mechanism (380) may include a solenoid that may be activated by signals generated by gauss detector (378) to drive a ram, rod, screw, and/or etc. into engagement with lock catch (390) to lock door (182). In other versions, lock mechanism (380) may include a motor or other electromechanical device configured to engage lock catch (390) and hold lock catch (390) in a given position. In still other versions, lock mechanism (380) may include a magnet, which may be activated by signals generated by gauss detector (378) to attract lock catch (390) and lock door (182). Of course, various alternative configurations for lock mechanism (380) may be apparent to those of ordinary skill in the art in view of the teachings herein.

As with lock catches (330, 360) described above, lock catch (390) may include features configured to promote locking of door (182) such as an attachment portion and a lock opening. As similarly discussed above, such features may permit attachment of lock catch (390) to door (182) or other components of control module (110). Such features may additionally permit engagement between lock catch (390) and lock mechanism (380) to hold lock catch (390) in a given position relative to lock body (372).

In some versions, lock catch (390) may be omitted entirely. For instance, in such versions, lock assembly (370) may be configured as an alarm-based lock rather than a physical lock. In such versions, lock mechanism (380) may not be used to physically lock door (182). Instead, lock mechanism (380) may be configured to detect the state of door (182) (e.g., open or closed) and then emit an alarm (auditory, visual, tactile, and/or etc.) if the state of door (182) changes when an electromagnetic field is detected by gauss detector (378). In one version, lock mechanism (380) may emit an alarm when door (182) is in an open state and an electromagnetic field is detected by gauss detector (378).

### V. Example Operational Modes for MRI Compatibility

In some circumstances, certain ways in which control module (110) is used may improve MRI compatibility of control module (110). For instance, certain hardware components may be susceptible to generating electromagnetic noise. Such electromagnetic noise may interfere with an MRI coil similar to MRI coil (154) described above. Thus, it may be desirable to incorporate certain operational modes into control module (110) to reduce electromagnetic noise during certain points in a biopsy procedure.

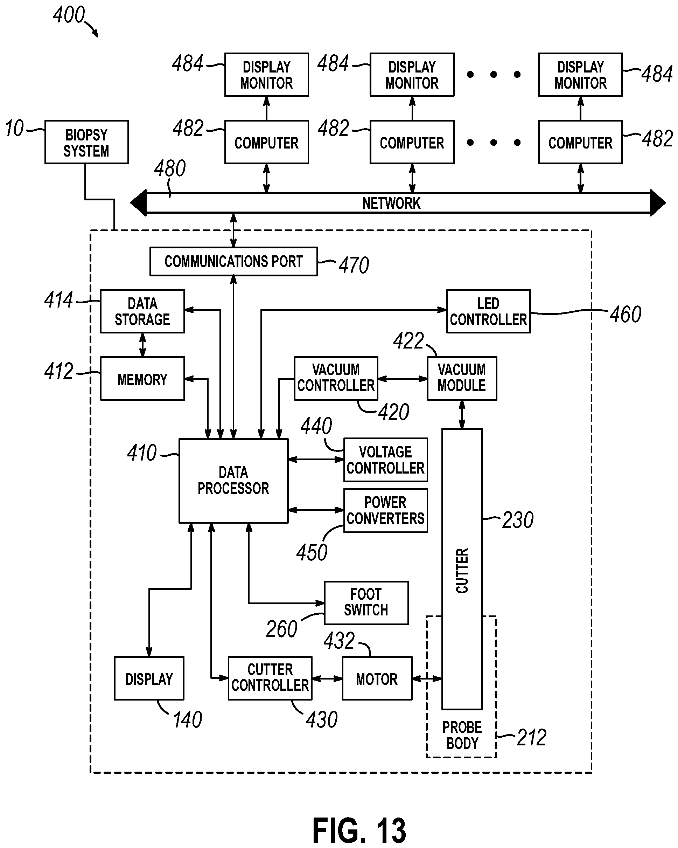

FIG. 13 shows a schematic diagram of an example of a signal processing system (400) that may be readily incorporated into biopsy system (10) described above. In particular, signal processing system (400) is generally configured to control functions of biopsy device (210) and control module (110). Although signal processing system (400) is shown as a single system used in connection with biopsy device (10) and vacuum control module (110), it should be understood signal processing system (400) may be divided into one or more separate systems dedicated to particular functions of biopsy device (210) and control module (110).

Signal processing system (400) includes a data processor (410) in communication with a plurality of controllers and/or interfaces (420, 430, 440, 450, 460) used to implement various functions of biopsy device (210) and control module (110). Data processor (410) is further in communication with memory (412) and data storage (414), which may be used in combination with data processor (410) to facilitate various functions of data processor (410) described in greater detail below. Although data processor (410) of the present example is shown as a single element, it should be understood that data processor (410) may include multiple processors in some examples, with one or more of such multiple processors being dedicated to particular functions.

Memory (412) may include random access memory (RAM), which may be configured for short-term storage of data. Meanwhile, data storage (414) may include a solid-state drive or a hard disk drive, or other suitable devices configured for long-term storage of data. Memory (412) and data storage (414) may also be in communication with each other to facilitate transfer of data between short-term storage and long-term storage. Together, data processor (410), memory (412), and data storage (414) may be configured to execute computer programs, in the form of software, to implement various functions of biopsy device (210) and/or control module (110) described in greater detail below. Although data processor (410), memory (412), and data storage (414) are shown as being in relatively close physical proximity, it should be understood that any one or more of data processor (410), memory (412), and data storage (414) may be physically separated from each other and one or more functions described herein may be performed remotely (e.g., cloud-based computation and signal processing).

In some examples, data processor (410), memory (412), and/or data storage (414) may be disposed within control module (110) and may communicate with other elements of biopsy system (10) via cable (112). In other examples, data processor (410), memory (412), and/or data storage (414) may be disposed within other components such as one or more portions of biopsy device (210). Such a configuration may be particularly desirable where data processor (410) is divided into multiple separate data processors (410) or sub-processors. In such examples, one or more data processors (410) may be disposed within one or more portions of biopsy device (210), while one or more data processors (410) may be disposed within control module (110). In such configurations, specific data processors (410) may be localized in connection with particular functions.

Signal processing system (400) further includes a vacuum controller (420) in communication with data processor (410). Vacuum controller (420) in combination with data processor (410) is generally configured to control various vacuum functions associated with biopsy device (210). Thus, vacuum controller (420) is in communication with vacuum module (422), which may be in communication with one or more of vacuum canister (120), valve port (130), and/or other components associated with control module (110) to facilitate delivery of vacuum, atmospheric air, and/or saline to biopsy device (210).

Signal processing system (400) further includes a cutter controller (430) in communication with data processor (410). Cutter controller (430) in combination with data processor (410) is generally configured to control various functions associated with movement of cutter (230) or other associated components. For instance, cutter controller (410) may be in communication with one or more motors (432) to communicate or receive signals to one or more motors (432) to drive one or more motors (432). One or more motors (432) may then drive movement of cutter (230) via cable (112) and associated drive components.

Signal processing system (400) further includes a voltage controller (430) a voltage controller (440) and one or more power converters (450). Voltage controller (440) and power converters (450) may be used in combination with data processor (410) to provide power to various components of control module (110) and/or biopsy device (210) with various characteristics (e.g., different voltages, currents, etc.). For instance, voltage controller (440) and/or power converters (450) may be in communication with any one or more of vacuum controller (420), cutter controller (430), and other components described herein to provide power suitable to operate such components on an as-needed basis. Thus, data processor (440) may be in communication with voltage controller (440) and power converters (450) to selectively apply suitable power to various components of control module (110) and/or biopsy device (210).

Signal processing system (400) further includes an light emitting diode (LED) controller (460). LED controller (460) is generally in communication with data processor (410) to provide control of one or more LEDs associated with control module (110). As will be described in greater detail below, such LEDs may be used to communicate certain operational states to an operator. Although LEDs may be used in the present version, other suitable operator communication components may be used in other versions such as buzzers, speakers, vibrators, tone generators, and/or etc. In still other versions, LEDs and LED controller (460) may be omitted entirely.

Data processor (410) may also be in communication with display (140) of control module (110). Optionally, a graphical processing module or graphical processing feature may be used in combination with display (140) and data processor (410) to drive display (140). Generally, data processor (410) may be configured to drive display (140) to communicate certain information to an operator. For instance, data processor (410) may drive display (140) to show graphical features associated with biopsy device (210) to illustrate operational conditions such as the position of cutter (230) relative to lateral aperture (226), the level of vacuum within needle assembly (220) and/or cutter (230), the status of tissue sample holder (240) with respect to tissue sample occupancy, the particular mode of operation of biopsy device (210), and/or etc. Additionally, data processor (410) may be configured to drive display (140) to show specific user interface screens associated with one or more operational modes of biopsy device (210) as will be described in greater detail below.

Data processor (410) may also be in communication with foot switch (260). Generally, data processor (410) may be configured to receive one or more user inputs from foot switch (260) to initiate one or more algorithums in response to such user inputs. By way of example only, depression of user input feature (266) may send a signal to data processor (410). Upon receiving the signal, data processor (410) may initiate a cutting sequence algorithm where vacuum controller (420) and cutter controller (430) are controlled by data processor (410) in sequence to sever a tissue sample using cutter (230) and transport the severed tissue sample through cutter (230) using vacuum controlled by vacuum module (420). Of course, various other algorithums may be initiated by communication of signals from foot switch (260) to data processor (410).

Signal processing system (400) further includes a communications port (470) in communication with data processor (410). As can be seen, communication port (470) may be used to couple signal processing system (400) with a network (480) such as a hospital picture archiving and communication system (PACS). Thus, communication port (470) may link signal processing system (400) to a plurality of remote computers (482) and displays (484) for remote viewing of data associated with biopsy system (10). Although the present example includes communications port (470), it should be understood that communications port (470) is optional in other examples and may be omitted.

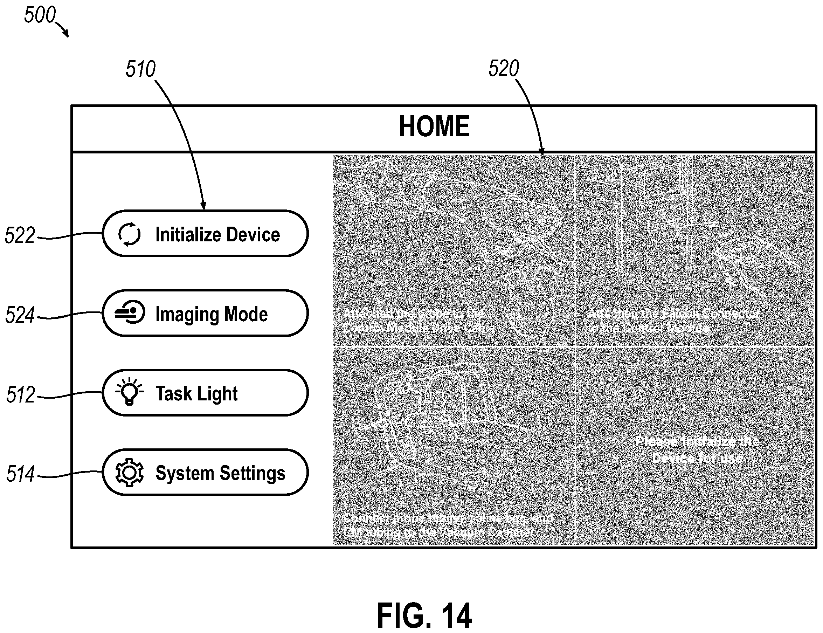

FIGS. 14 shows an example of a home screen (500) that may be communicated to an operator by display (140) via data processor (410). Home screen (500) is generally configured to provide an initial operational interface or landing page. From home screen (500), an operator may select an operational mode from an array of operational modes (510), activate one or more features using a toggle graphic (512), or adjust system settings using a menu button (514). Additionally, home screen (500) may include an instruction window (520), which may include one or more graphical instructions for setting up biopsy system (10).

Array of operational modules (510) of the present version includes two buttons (522, 524) for selection of a given operational mode. For instance, button (522) may initiate an initialization mode, which may be used to prepare biopsy device (210) for use in a biopsy procedure. Additionally, button (524) may initiate an imaging mode. As will be described in greater detail below, imaging mode may initiate an imaging mode algorithm, which may be used to put control module (110) in an operational state for limited interference with an MRI coil such as MRI coil (152) described above.

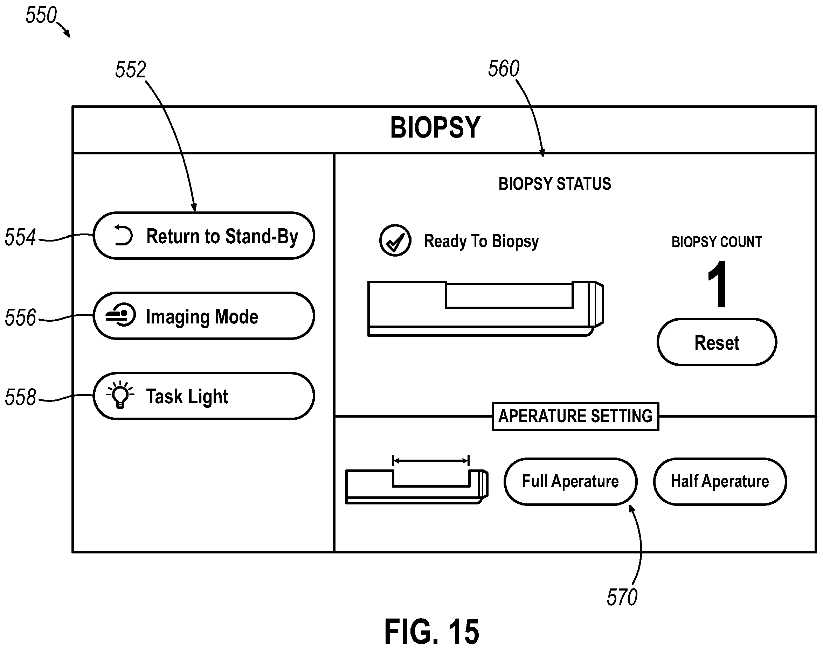

FIG. 15 shows an example of a biopsy screen (550) that may be communicated to an operator by display (140) via data processor (410). Biopsy screen (550) is generally configured for use during a biopsy procedure to facilitate operation of biopsy device (210). In particular, biopsy screen (550) may include a mode pane (552), a biopsy status pane (560), and an aperture setting pane (570). Panes (552, 560, 570) are generally configured to group various common user interface functions in particular groupings. Although certain specific panes (552, 560, 570) are shown in the present example, the particular panes (552, 560, 570) shown are merely optional and any one or more of panes (552, 560, 570) may be omitted in some versions.

Mode pane (552) is generally configured to permit an operator to toggle various modes of operation using one or more buttons (554, 556, 558). For instance, mode pane (552) includes a standby button (554), which permits an operator to initiate control module (110) entering a standby mode. Similarly, mode pane (552) further includes an imaging mode button (556), which may initiate the imaging mode. As will be described in greater detail below, imaging mode may initiate the imaging mode algorithm, which may be used to put control module (110) in an operational state for limited interference with an MRI coil such as MRI coil (152) described above. Finally, mode pane (552) includes an optional task light button (558), which may be used to activate a task light integrated into a portion of control module (110) or display (140) itself when such a task light is included within control module (110).

Biopsy status pane (560) may be configured to provide various status information related to biopsy device (210). For instance, biopsy status pane (560) may include a graphical indicator showing the status of needle assembly (220) and/or cutter (230). Biopsy status pane (560) may further include an optional counter that may be configured to automatically update each time a biopsy sample is collected using biopsy device (210). In some versions, the counter may include a reset button to permit manual resetting.

Aperture setting pane (570) may be configured to provide variable aperture functionality. For instance, aperture setting pane (570) may include a graphical representation of needle assembly (220) and cutter (230), which may show the relative position of cutter (230) within lateral aperture (226). Aperture setting pane (570) may also include one or more buttons to adjust the distal retraction position of cutter (230), effectively changing the size of lateral aperture (226). In the present version, aperture setting pane (570) may permit adjustment between a full aperture position and a half aperture position. In other versions, aperture setting pane (570) may include other settings such as quarter aperture, three quarter aperture, and/or etc.

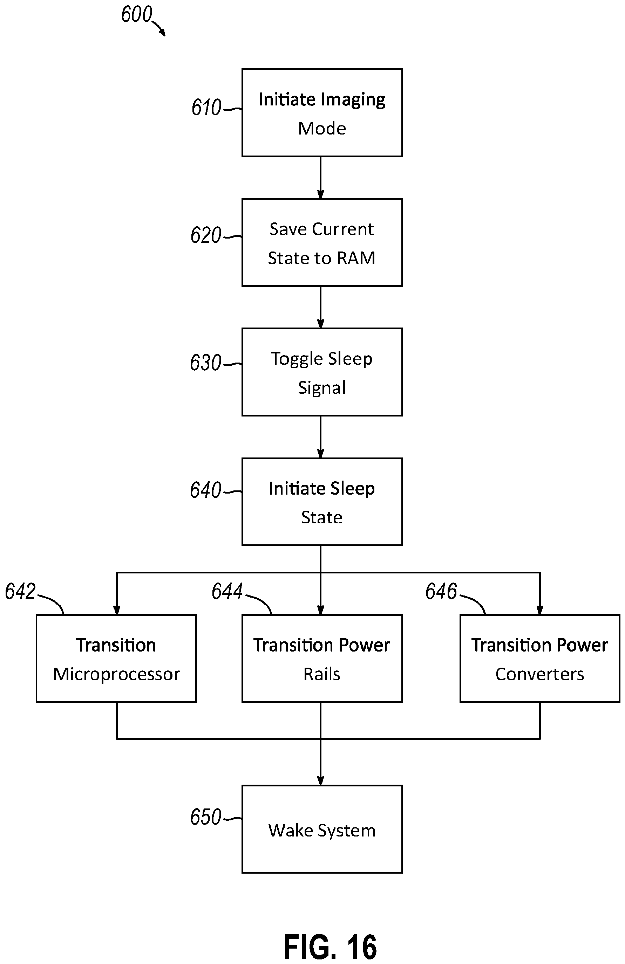

FIG. 16 shows an example of an imaging mode algorithm (600) that may be initiated by control module (110) using data processor (410) with input from display (140). Imaging mode is generally configured to reduce the electromagnetic footprint of control module (110) so that control module (110) may be positioned within a portion of an MRI suite proximate to an MRI coil without interference with components of the MRI coil during operation of the MRI coil. As will be described in greater detail below, such a reduction in electromagnetic footprint may be achieved by data processor (410) adjusting operations of data processor itself and various controllers and/or interfaces (420, 430, 440, 450, 460) to reduce electromagnetic noise generated by control module (110).

As described above, imaging mode may be initiated by an operator pressing any one of buttons (524, 556) on home screen (500) or biopsy screen (550). Initiation of imaging mode is shown in FIG. 16 at block (610). Although not shown, it should be understood that other user interface screens may also include a button configured to activate imaging mode. The presence of a dedicated imaging mode button on multiple user interface screens is generally desirable to facilitate initiation of imaging mode at a variety of procedural steps during a biopsy procedure such as during setup of control module (110) and biopsy device (210), or during use of control module (110) and biopsy device (210) during a biopsy procedure. As will be appreciated, it may be desirable to initiate imaging mode at a variety of points during a biopsy procedure depending on when an MRI coil is in use. Thus, having a button configured to initiate imaging mode on multiple screens may facilitate ease of use, as well as provide a visual reminder to an operator to enter imaging mode.

Once imaging mode is initiated, the current state of control module (110) may be saved to memory as shown in block (620). In particular, data processor (410) may communicate with memory (412) to save the current state of control module (110) to RAM. Alternatively, in some versions, data processor (410) may instead communicate with data storage (414) to save the current state of control module (110) to long term memory. As will be described in greater detail below, saving of this current state may facilitate waking of control module (110) once imaging mode is deactivated.

Optionally, initiation of imaging mode can include a vent vacuum operation either before saving the current state of control module (110) to memory or after saving the current state. In some examples, the vent vacuum operation alternatively occurs immediately upon an operator pressing buttons (524, 556) to enter imaging mode. Regardless, the vent vacuum operation includes cycling valve assembly (113) to vent needle assembly (220) and or other portions of biopsy device (210). In such a cycle, venting is applied for a predetermined time. Suitable predetermined times include, for example, 8 seconds, 8 or more seconds, 5 to 10 sections, 5 to 15 seconds, and/or etc. Regardless of the particular predetermined time, such venting is generally configured to vent the majority of vacuum pressure from the system. It should be understood that in some uses, vacuum pressure may not be present in the system upon initiation of imaging mode (e.g., initiation of imaging mode before any biopsy has been performed). Thus, in some examples, the vent vacuum operation is only initiated from certain specific screes such as biopsy screen (550) using button (556), while other screens such as home screen (500) will omit the vent vacuum operation upon pressing button (524).

After the current state of control module (110) is saved, data processor (410) may toggle a sleep signal as shown in block (630). The presence of the sleep signal shown in block (630) may then initiate a sleep state within various components of control module (110) as shown in block (640).

Initiation of the sleep state shown at block (640) may initiate shut off or reduce the functionality of various features of control module (110) as shown in blocks (642, 644, 646). In particular, as can be seen at block (642), data processor (410) itself may change from an active state to a reduced function state. In the active state, data processor (410) may be configured to operate at the normal operating frequency of data processor (410) (e.g., about 1.8 GHz or more). By contrast, in the reduced function state, the operating frequency of data processor (410) may be substantially reduced. In the present version, the operating frequency is reduced to about 32.768 kHz (about 32 kHz), or about 30 to 40 kHz in some versions. However, other reduced operating frequencies may be used such as 50 kHz or less. Generally, the particular reduction in operating frequency is sufficient to render operation of data processor (410) at a frequency below the operation of MRI coils (e.g., 1MHz to 300MHz). As a result, any electromagnetic radiation generated by data processor (410) may not interfere with the MRI coil and be less susceptible to generating image artifacts.

As shown in block (644), initiation of the sleep state may also include modifying the operation of voltage controller (440). In particular, the processor voltage board may remain at 12 volts. However, the power rails for electromechanical systems may be powered off. For instance, power to vacuum controller (420), vacuum module (422), cutter controller (430), and/or etc. may be powered off. Additionally, as shown at block 646), power converters (450) may also be shutoff.

By powering off such electromechanical systems, electromagnetic radiation generated by control module may be reduced. For instance, typical electronic and electromechanical control systems may include a plurality of clocks and conversion frequencies that may generate radio frequency noise. Such noise may be incidentally within the same range that typical MRI coils operate within (e.g., 1MHz to 300MHz). Thus, by powering off such electromechanical systems, control module (110) may produce less noise, particularly within the frequency range used for operation of MRI coils.

Once the sleep state is achieved as shown in blocks (642, 644, 646), only some components may remain active. For instance, as described above, data processor (410) may remain partially active. Power may still also be supplied to data processor (410) at 12 volts. Additionally, LED controller (460) may remain active. In particular, LED controller (460) may remain active to provide a power button indicator oriented around or proximate to power button (142). For instance, when control module is in the sleep state, LED controller (460) may be used to control LEDs to provide a visual indicator (e.g., pulsing) that power button (142) may be used to wake control module (110). In some examples, only power is provided to the LEDs associated with power button (142). Meanwhile, control of such LEDs is provided by discrete logic.

The sleep state may be interrupted as shown in block (650). In particular, the sleep state may be interrupted by pressing power button (142). Once power button (142) is pressed, data processor (410) may receive a wake signal. This wake signal may cause data processor (410) to first return to normal operating frequency. Data processor may then return voltage controller (440) and power converters (450) to their active states. This may permit vacuum controller (420), vacuum module (422) and cutter controller (430) to all return to their active states. Finally, data processor (410) may retrieve the previously saved current state from memory (412) or data storage (414) and cause control module (110) to return to the operational state immediately prior to entering imaging mode.

Although imaging mode described above may be activated or deactivated using a user input such as an input from graphical buttons (524, 556) and/or physical button (142), imaging mode may be activated or deactivated by other means in some versions. For instance, data processor (410) may be configured to implement a timeout counter. This timeout counter may be used to automatically initiate imaging mode when no activity is detected after a predetermined period of time. Such an automatic initiation of imaging mode may be desirable to prevent imaging artifacts even when an operator inadvertently fails to enter imaging mode. In addition, or in the alternative, such a timeout counter may be used to automatically shutoff control module (110) when no activity is detected after a predetermined period of time. Such an automatic shutoff may be desirable to prevent battery drain. The particular predetermined time for automatic shutoff may be varied, but in some versions may be a function of the time typically needed to conduct MRI imaging.

Although imaging mode is described above in the context of reduction of imaging artifacts during imaging, it should be understood that one or more aspects of imaging mode can be readily used in other circumstances. For instance, in some examples, imaging mode as described above, can be used for power savings regardless of whether imaging is actively being performed. Power savings may be desirable where control module (110) is battery powered to conserve battery power for functions such as collecting biopsy samples. Thus, in some circumstances, imaging mode can be referred to as a sleep function, which can be activated at any time control module (110) is not in active use either automatically, with user input, or some combination thereof.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A control module for use in a magnetic resonance imaging (MRI) guided biopsy procedure using an MRI coil, the control module comprising: a body, including: one or more ports configured to couple the control module to a biopsy device, and a display configured to output one or more biopsy device status indicators; and a plurality of MRI compatibility features, the MRI compatibility features being configured to reduce an electromagnetic footprint of the control module.

### Example 2

The control module of Example 1, the plurality of MRI compatibility features including one or more data processors, the one or more data processors being configured to initiate an imaging mode.

### Example 3

The control module of Example 1, the plurality of MRI compatibility features including one or more data processors, the one or more data processors being configured to: initiate an imaging mode; power off one or more electromechanical control systems in response to initiation of the imaging mode; and enter a reduced function mode in response to initiation of the imaging mode.

### Example 4

The control module of Example 3, the one or more data processors having an operational frequency of about 30 to 40kHz in the reduced function mode.

### Example 5

The control module of any of Examples 3 or 4, the one or more data processors being further configured to initiate the imaging mode in response to a user input.

### Example 6

The control module of any of Examples 3 through 5, the one or more data processors being further configured to initiate the imaging mode after no activity associated with the control module is detected for a predetermined period of time.

### Example 7

The control module of any of Examples 1 through 7, the plurality of MRI compatibility features including a control module structural layout, the control module structural layout being configured with high-density ferrous components oriented proximate a bottom portion of the control module.

### Example 8

The control module of Example 7, the high-density ferrous components including one or more motors or one or more vacuum pumps.

### Example 9

The control module of any of Examples 7 or 8, the control module structural layout further including one or more linkages, the one or more linkages being configured to communicate rotary power from a lower portion of the control module to an upper portion of the control module.

### Example 10

The control module of any of Examples 7 through 9, the control module structural layout further including a non-magnetic metal base, a plurality of non-magnetic metal structural members extending upwardly from the base, and a plurality of non-magnetic body panels disposed between the plurality of structural members.

### Example 11

The control module of any of Examples 1 through 10, the plurality of MRI compatibility features further including a rotary drive cable, the rotary drive cable being configured to communicate mechanical power to the biopsy device, the rotary drive cable defining a length, the length being about two times the height of the control module or less.

### Example 12

The control module of Example 11, the rotary drive cable including phosphor bronze.

### Example 13

The control module of any of Examples 1 through 12, the plurality of MRI compatibility features including the control module being configured to operate using direct current.

### Example 14

The control module of any of Examples 9 through 13, the plurality of MRI compatibility features including a battery compartment and a battery received within the battery compartment, the battery compartment including a door and a lock mechanism, the lock mechanism being configured to lock the door to thereby enclose the battery within the battery compartment.

### Example 15

The control module of Example 1, the lock mechanism being configured to lock the door automatically in response to the presence of an electromagnetic field.

### Example 16

A biopsy system for performing a magnetic resonance imaging (MRI) guided biopsy procedure within an MRI suite, the MRI suite including an MRI coil, the system comprising: a biopsy device including a probe, the probe including probe body, a needle extending from the probe body, and a cutter movable relative to the needle to sever one or more tissue samples; a control module, the control module including one or more motors, the one or more motors being oriented proximate a base of the control module; and a cable extending from the control module to the biopsy device, the cable being configured to communicate power from the control module to the biopsy device.

### Example 17

The system of Example 16, the biopsy device including only non-magnetic materials.

### Example 18

The system of Examples 16 or 17, further comprising a foot switch, the foot switch being in communication with the control module to control one or more functions of biopsy device via the control module, the foot switch including only MRI compatible materials.

### Example 19

The system of any of Examples 16 through 18, the control module further including a data processor and a motor controller in communication with the one or more motors, the data processor being configured to initiate an imaging mode, the data processor being further configured to disable the motor controller upon initiation of the imaging mode.

### Example 20

The system of any of Examples 16 through 18, the control module further including a display, a data processor in communication with the display, and a plurality of controllers in communication with the data processor, the display being configured to depict a plurality of user interface screens, each user interface screen of the plurality of user interface screens including an imaging mode button, the imaging mode button of each user interface screen being configured to provide an input to the data processor to initiate an imaging mode, the data processor being configured to disable one or more controllers of the plurality of controllers when initiating the imaging mode.

### Example 21

The system of Example 20, the data processor being further configured to transition from an active mode to a reduced function mode when initiating the imaging mode, the data processor having an active operating frequency corresponding to the active mode, the data processor having a reduced function operating frequency corresponding to the reduced function mode, the reduced function operating frequency being less than the active operating frequency.

### Example 22

A method of increasing magnetic resonance imaging (MRI) compatibility of a control module of a biopsy system for use of the control module proximate to an MRI coil, comprising: initiating an imaging processing mode by communicating a signal to a data processor; saving a current state of the control module to memory; generating a sleep signal using the data processor; and initiating a sleep state within the control module by fully or partially disabling one or more components of the control module.

### Example 23

The method of Example 22, the step of initiating the sleep state including transitioning the data processor from an active state to a reduced function state.

### Example 24

The method of Examples 23, the reduced function state of the data processor having an operating frequency less than an operating frequency of the active state.

### Example 25

The method of any of Examples 22 through 24, the step of initiating the sleep state including disabling one or more power rails in communication with one or more controllers.

### Example 26

The method of any of Examples 22 through 24, the step of initiating the sleep state including disabling a power rail in communication with a cutter controller and disabling a power rail in communication with a vacuum controller.

### Example 27

The method of any of Examples 22 through 26, the step of initiating the sleep state including disabling one or more power converters.

### Example 28

The method of any of Examples 22 through 27, further comprising communicating a wake signal to the data processor, and enabling the one or more components of the control module disabled or partially disabled during the step of initiating the sleep state.

### Example 29

The method of any of Examples 22 through 28, further comprising powering off the control module after detection of inactivity of the control module for a predetermined period of time.

### VI. Conclusion

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A control module for use in a magnetic resonance imaging (MRI) guided biopsy procedure using an MRI coil, the control module comprising:
(a) a body, including:
(i) one or more ports configured to couple the control module to a biopsy device, and
(ii) a display configured to output one or more biopsy device status indicators; and
(b) a plurality of MRI compatibility features, the MRI compatibility features being configured to reduce an electromagnetic footprint of the control module.

2. The control module of claim 1, the plurality of MRI compatibility features including one or more data processors, the one or more data processors being configured to initiate an imaging mode.

3. The control module of claim 1, the plurality of MRI compatibility features including one or more data processors, the one or more data processors being configured to:
initiate an imaging mode;
power off one or more electromechanical control systems in response to initiation of the imaging mode; and
enter a reduced function mode in response to initiation of the imaging mode.

4. The control module of claim 3, the one or more data processors having an operational frequency of about 30 to 40kHz in the reduced function mode.

5. The control module of any of claims 3 or 4, the one or more data processors being further configured to initiate the imaging mode in response to a user input.

6. The control module of any of claims 3 through 5, the one or more data processors being further configured to initiate the imaging mode after no activity associated with the control module is detected for a predetermined period of time.

7. The control module of any of claims 1 through 7, the plurality of MRI compatibility features including a control module structural layout, the control module structural layout being configured with high-density ferrous components oriented proximate a bottom portion of the control module.

8. The control module of claim 7, the high-density ferrous components including one or more motors or one or more vacuum pumps.

9. The control module of any of claims 7 or 8, the control module structural layout further including one or more linkages, the one or more linkages being configured to communicate rotary power from a lower portion of the control module to an upper portion of the control module.

10. The control module of any of claims 7 through 9, the control module structural layout further including a non-magnetic metal base, a plurality of non-magnetic metal structural members extending upwardly from the base, and a plurality of non-magnetic body panels disposed between the plurality of structural members.

11. The control module of any of claims 1 through 10, the plurality of MRI compatibility features further including a rotary drive cable, the rotary drive cable being configured to communicate mechanical power to the biopsy device, the rotary drive cable defining a length, the length being about two times the height of the control module or less.

12. The control module of claim 11, the rotary drive cable including phosphor bronze.

13. The control module of any of claims 1 through 12, the plurality of MRI compatibility features including the control module being configured to operate using direct current.

14. The control module of any of claims 9 through 13, the plurality of MRI compatibility features including a battery compartment and a battery received within the battery compartment, the battery compartment including a door and a lock mechanism, the lock mechanism being configured to lock the door to thereby enclose the battery within the battery compartment.

15. The control module of claim 1, the lock mechanism being configured to lock the door automatically in response to the presence of an electromagnetic field.
